(19) Europäisches Patentamt
European Patent Office
Office européen des brevets

(11) Publication number: **0 038 061**
**B1**

(12) **EUROPEAN PATENT SPECIFICATION**

(45) Date of publication of patent specification: **23.01.85**

(21) Application number: **81102810.9**

(22) Date of filing: **13.04.81**

(51) Int. Cl.[4]: **C 07 C 47/55,** C 07 C 47/575, C 07 C 45/00

(54) **Substituted biphenyl-2-carboxaldehydes and a process for preparing them.**

(30) Priority: **14.04.80 US 140321**

(43) Date of publication of application:
**21.10.81 Bulletin 81/42**

(45) Publication of the grant of the patent:
**23.01.85 Bulletin 85/04**

(84) Designated Contracting States:
**AT BE CH DE FR GB IT LI LU NL SE**

(56) References cited:
**DE-A-2 801 887**
**DE-B-1 156 776**
**GB-A-1 499 655**

(73) Proprietor: **MERCK & CO. INC.**
**126, East Lincoln Avenue P.O. Box 2000**
**Rahway New Jersey 07065 (US)**

(72) Inventor: **Hoffman, William F.**
**740 Weikel Road**
**Lansdale Pennsylvania 19446 (US)**

(74) Representative: **Abitz, Walter, Dr.-Ing. et al**
**Abitz, Morf, Gritschneder P.O. Box 86 01 09**
**D-8000 München 86 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 038 061 B1

**Description**

This invention relates to substituted biphenyl-2-carboxaldehydes selected from 3,5-dimethyl-4′-fluoro-1,1′-biphenyl-2-carboxaldehyde; 3,3′,5,5′-tetramethyl-1,1′-biphenyl-2-carboxaldehyde; 3′,4′-dichloro-3,5-dimethyl-1,1′-biphenyl-2-carboxaldehyde; 5-chloro-4′-fluoro-3,3′-dimethyl-1,1′-biphenyl-2-carboxaldehyde; and 3,3′,4′,5-tetrachloro-1,1′-biphenyl-2-carboxaldehyde, and to a process for preparing the same. The substituted biphenyl-2-carboxaldehydes are needed as intermediates for synthetic, *in vivo* cholesterol synthesis inhibitors. The process for preparing the biphenyl-2-carboxaldehydes comprises reacting a palladium complex of the structure:

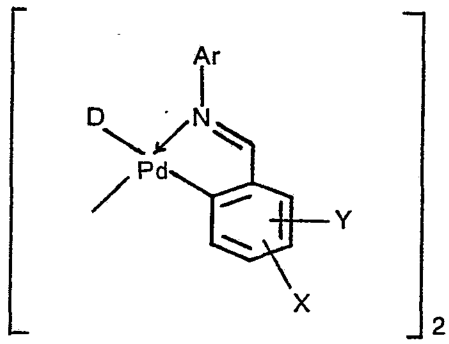

in which Ar is an insert aryl residue and D is an anion such as acetate with a Grignard reagent of the structure:

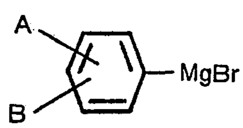

or the corresponding substituted phenyllithium in the presence of six to eight, preferably eight, molar equivalents of triphenylphosphine, wherein in the above formulas the substituents A, B, X and Y correspond to the substituents of the biphenyl-2-carboxaldehydes as defined above.

It is known that certain mevalonate derivatives inhibit the biosynthesis of cholesterol, cf. F. M. Singer et al., *Proc. Soc. Exper. Biol. Med., 102* 270 (1959) and F. H. Hulcher, *Arch. Biochem. Biophys., 146* 422 (1971). Nevertheless the activity of the known compounds has not always been found satisfactory, i.e. to have practical application.

Recently, Endo et al. reported (U.S. Letters Patent 4,049,495; 4,137,322; and 3,983,140) the production of a fermentation product which was quite active in the inhibition of cholesterol biosynthesis. This natural product, now called compactin, was reported by Brown et al. (*J. Chem. Soc.* Perkin I, 1165 (1976)) to have a complex mevalonolactone structure. However, the low rate of production from fermentation broths appears to limit the supply of this natural product.

More recently, Willard et al. (European patent application 80 104 807.5, Publication Number 0 024 348) described synthetic analogs of Endo's fermentation product whose biological activity closely approximated the natural product. As the structure-activity relationship within the Willard et al. compounds was developed, the desirable structures were seen to be derived especially from 2,4-dichloro-6-substituted benzaldehydes or 2,4-dimethyl-6-substituted benzaldehydes in which the 6-substituent was a substituted phenyl.

3,5-dichloro-4′-fluoro-1,1′-biphenyl-2-carboxaldehyde is prepared by Willard et al. using a Grignard reagent.

Murahashi et al. (*J. Org. Chem., 43* 4099—4106 (1978) and Tetrahedron Letters 3749 (1974)) has described a method of preparing ortho-substituted benzaldehydes which comprises reacting a cyclic palladium complex of benzaldehyde aniline Schiff bases with a Grignard reagent in the presence of four molar equivalents of triphenylphosphine. When this reaction was tried with the substituted benzaldehydes and Grignard reagents having substituents needed for the Willard et al. inhibitors, an unusable mixture of various aldehydes was obtained.

According to the process of the invention six to eight molar equivalents of triphenylphosphine are used to prepare the new substituted biphenyl-2-carboxaldehydes as defined above. However, the invention is related to the surprising properties of the present compounds, rather than to their preparation. It has been found that the reductase inhibitors prepared from the compounds of the present invention show a surprisingly outstanding better effect than the best inhibitor known from European patent application 80 104 807.5.

According to the claimed process the desired biphenyl-2-carboxaldehydes are obtained in 75—85% yields. This synthesis is shown in Flow Sheet A. While the above formula describes a Schiff base

of aniline, where Ar is phenyl, other aromatic amines having unreactive substituents such as toluidine and xylidine can obviously be used.

FLOW SHEET A

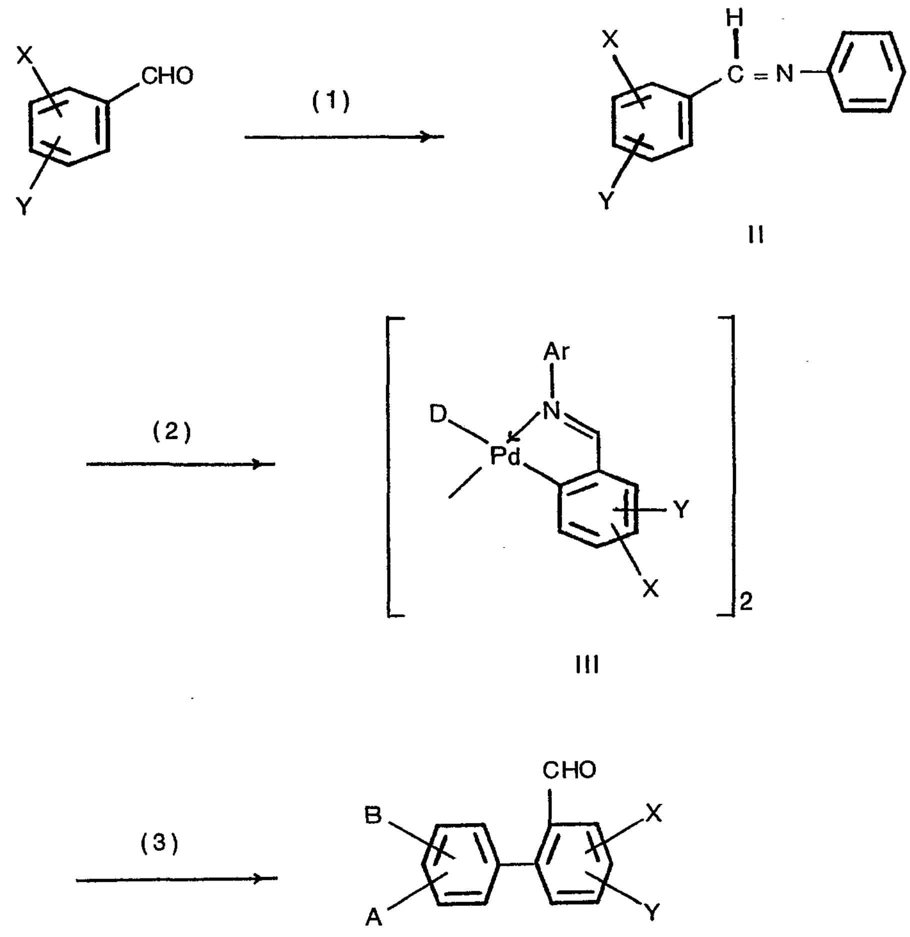


II

III

IV

Equivalents:

A, B, Y and X as defined D is an anion such as chloride or acetate.

Reactions:

(1) Reaction with aniline or other aromatic amine.

(2) Reaction with a palladium salt in the presence of an acid, e.g. palladium acetate and acetic acid.

(3) Reaction with a Grignard reagent:

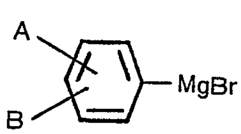

or the corresponding substituted phenyllithium in the presence of six to eight molar equivalents of triphenylphosphine in a suitable solvent such as benzene or toluene.

The biphenyl-2-carboxaldehydes of this invention are useful for the synthesis of compounds of structure X in Flow Sheet B by the process described in Flow Sheet B and the Reactions appended thereto.

The compounds, X, are potent inhibitors of HMG-CoA-reductase and hence useful as antihypercholesterolemic agents in the treatment of hypercholesterolemia in human or animal patients. A typical human adult dose is between 200 and 2,000 mg/day, which can be administered in a single dose or in divided doses given 1—4 times per day.

FLOW SHEET B

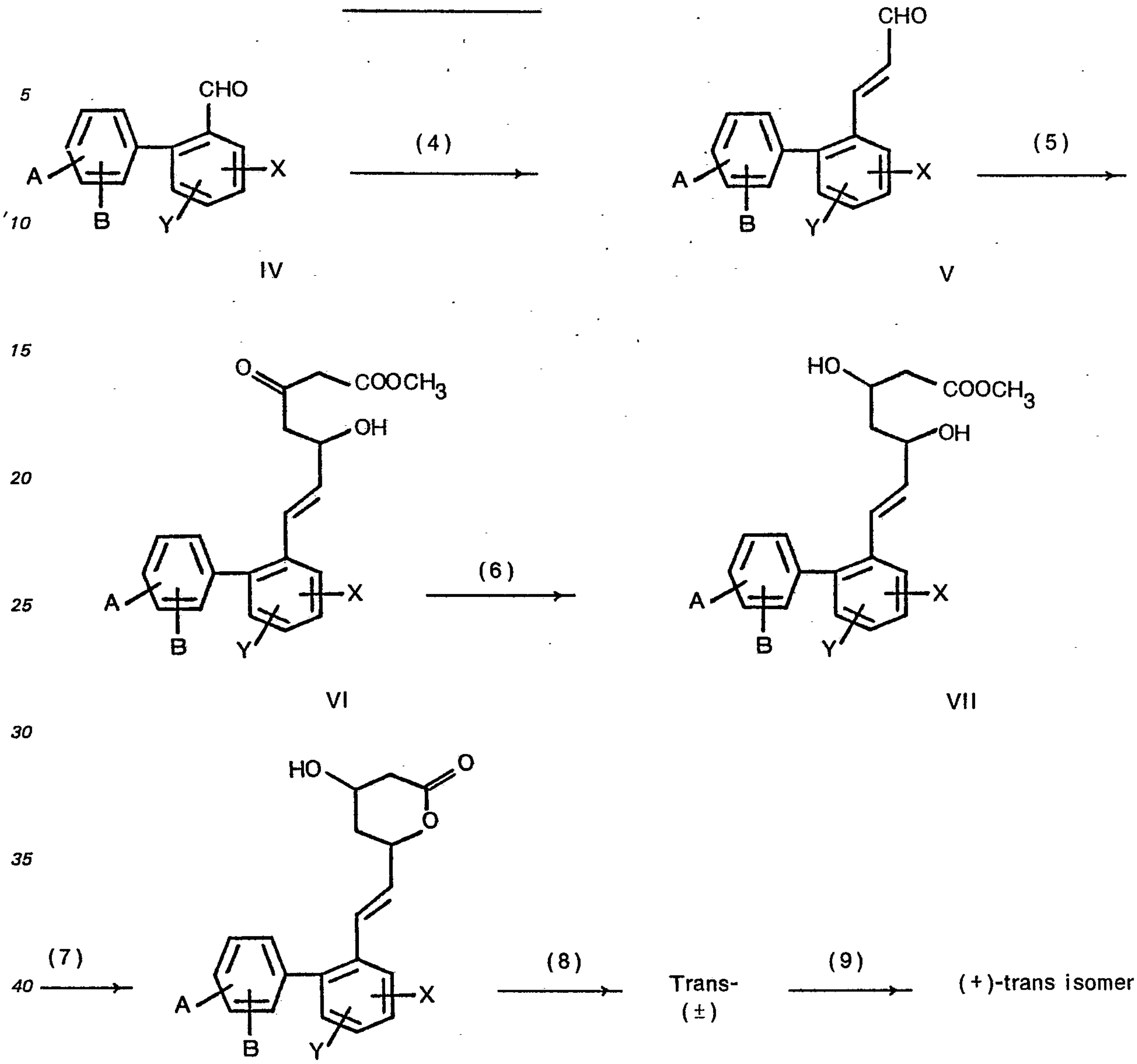


IV V VI VII VIII IX X

Reactions:

(4) Aldol reaction, e.g. either condensation of acetaldehyde with the benzaldehyde, followed by acetylation and thermal elimination of acetic acid, or a directed aldol condensation in which the anion of an N-substituted ethylidenylimine is condensed with the starting aldehyde, followed by dehydration and imine hydrolysis, or by reaction with 2-ethoxyvinyllithium followed by rearrangement.

(5) Reaction with the dianion of acetoacetic ester.

(6) Reduction with $NaBH_4$ at 0°C.

(7) Saponification by base, followed by lactonization in toluene.

(8) Separation of cis- and trans- isomers.

(9) Resolution.

Example 1

3,3′,4′,5-Tetrachloro-1,1′-biphenyl-2-carboxaldehyde

Step A:

Preparation of bis($\mu$-(acetato-O:O′)bis-[3,5-dichloro-2-[(phenylimino)methyl]phenyl-C,N]dipalladium

A mixture of N-[(2,4-dichlorophenyl)-methylene]benzene amine (2.5 g, 10 mmole) and palladium (II) acetate (2.24 g, 10 mmole) in acetic acid (50 ml) was heated at reflux for one hour with stirring. The warm turbid solution was filtered and the filtrate was diluted with water (300 ml) to give the title compound as a red solid (3.9 g, 94%). Crystallization from acetic acid-water (7:1, v:v) provided an analytical sample of the title compound, m.p. 203—5°C: pmr ($CDCl_3$) $\delta$ 1.73 (3H, s), 6.50 (H, d, J=1.5 Hz), 6.97 (2H, m), 7.12 (H, d, J=7.5 Hz), 7.33 (3H, m), 8.03 (H, s).

Analysis Calc. for $C_{30}H_{22}Cl_2N_4O_4Pd_2$
Calc.: C, 43.42 H, 2.67 N, 3.38
Found: C, 43.54 H, 2.59 N, 3.13

Step B:
Preparation of 3,3',4',5-Tetrachloro-1,1'-biphenyl-2-carboxyaldehyde

A solution of bis($\mu$-(acetato-0:0')bis[3,5-dichloro-2-[(phenylimino)methyl]phenyl-C,N]di-palladium (8.29 g, 10 mmole) and triphenylphosphine (21.0 g, 80 mmole) in dry benzene (150 ml) was stirred for 30 minutes at ambient temperature under $N_2$. 3,4-Dichlorophenylmagnesium bromide, prepared from 1-bromo-3,4-dichlorobenzene (88 mmole) and magnesium (80 mmole) in dry ether (100 ml) under $N_2$ at ambient temperature, was added to the above solution in one portion. The resultant mixture was stirred for 1 hour at ambient temperature. After the addition of 6 NHCl (50 ml) with stirring for 1 hour, the mixture was filtered. The filtrate was diluted with ether (300 ml) and washed with brine (2×100 ml). The organic layer was refiltered to remove more yellow solid and the filtrate was washed with brine (2×100 ml), dried over $MgSO_4$, filtered and evaporated. The residue was chromatographed on a silica column (1000 g). Elution with ether-hexane (1:39, v:v; 5500 ml) provided a forerun which was discarded. Continued elution with ether-hexane (1:9, v:v, 5700 ml) gave the title compound; pmr ($CDCl_3$) $\delta$ 7.0—7.6 (5H, m), 10.3 (H, s).

Example 2
5-Chloro-4'-fluoro-3,3'-dimethyl-1,1'-biphenyl-2-carboxaldehyde
Step A:
Preparation of N-[(4-chloro-2-methylphenyl)-methylene]benzeneamine

A mixture of 4-chloro-2-methylbenzaldehyde (3.5 g, 22.6 mmol) and aniline (2.11 g, 22.6 mmol) in toluene (40 ml) was heated at reflux in a Dean-Stark apparatus for 1 hour. The mixture was cooled and evaporated *in vacuo* to leave an oily residue. The residue was redissolved in ether and washed with 5% sodium bicarbonate solution. The organic phase was separated, dried over $MgSO_4$ and filtered. The filtrate was concentrated *in vacuo* to afford an oily residue which was purified by distillation via a Kugelrohr apparatus (oven temperature 160°C, 0.5 mm) to provide the title compound (4.0 g, 17.4 mmol, 77%) as a viscous oil; pmr ($CDCl_3$) $\delta$ 2.5 (3H, s), 6.3—7.5 (7H, m), 7.95 (H, d), 8.6 (H, s).

Step B:
Preparation of 5-Chloro-4'-fluoro-3,3'-dimethyl-1,1'-biphenyl-2-carboxaldehyde

By substituting an equimolar amount of N-[(4-chloro-2-methylphenyl)methylene]benzeneamine in place of N-[(2,4-dichlorophenyl)methylene]benzeneamine in Step A of Example 1 and replacing the 3,4-dichlorophenylmagnesium bromide with an equimolar amount of 4-fluoro-3-methyl-phenylmagnesium bromide in Step B of Example 1 and following the procedures described therein, there was obtained a corresponding amount of the title compound; pmr ($CDCl_3$) $\delta$ 2.30 (3H, d), 2.60 (3H, s), 7.1—7.3 (5H, m), 9.9 (H, s).

Example 3
3',4'-Dichloro-3,5-dimethyl-1,1'-biphenyl-2-carboxaldehyde

By substituting an equimolar amount of N-[(2,4-dimethylphenyl)methylene]benzeneamine in place of N-[(2,4-dichlorophenyl)methylene]benzeneamine in Step A of Example 1 and replacing the 3,4-dichlorophenylmagnesium bromide with an equimolar amount of 3,4-dichlorophenylmagnesium bromide in Step B of Example 1 and following the procedures described therein, there was obtained a corresponding amount of the title compound, m.p. 80—81°C; pmr ($CDCl_3$) $\delta$ 2.4 (3H, s), 2.6 (3H, s), 7.0—7.5 (5H, m), 10.0 (H, s).

Analysis calc. for $C_{15}H_{12}Cl_2O$: C, 64.54 H, 4.33
Found: C, 64.83 H, 4.45

Example 4

Employing the procedure substantially as described in Example 3, but substituting for the 3,4-dichlorophenylmagnesium bromide used therein, an equimolecular amount of the Grignard reagents listed in Table II there were prepared the substituted biphenyl-2-carboxaldehydes, also listed in Table II.

| Grignard | Product | pmr ($\delta$) |
|---|---|---|
| 3,5-dimethylphenyl | 3,3',5,5'-tetramethyl-1,1'-biphenyl-2-carboxaldehyde | 2.3 (9H, m), 2.6 (3H, s) 6,8—7,0 (5H, m), 10.0 (H, s) |
| 4-fluorophenyl | 3,5-dimethyl-4'-fluoro-1,1'-biphenyl-2-carboxaldehyde | 2.4 (3H, s), 2.6 (3H, s) 7.0—7.4 (6H, m), 10.0 (H, s) |

**Claims for the Contracting States: BE, CH, DE, FR, GB, IT, LI, LU, NL, SE:**

1. A compound selected from: 3,5-dimethyl-4′-fluoro-1,1′-biphenyl-2-carboxaldehyde; 3,3′,5,5′-tetramethyl-1,1′-biphenyl-2-carboxaldehyde; 3′,4′-dichloro-3,5-dimethyl-1,1′-biphenyl-2-carboxaldehyde; 5-chloro-4′-fluoro-3,3′-dimethyl-1,1′-biphenyl-2-carboxaldehyde; 3,3′,4′,5-tetrachloro-1,1′-biphenyl-2-carboxaldehyde.

2. Process for preparing a biphenyl-2-carboxaldehyde according to claim 1, which comprises reacting a palladium complex of the structure:

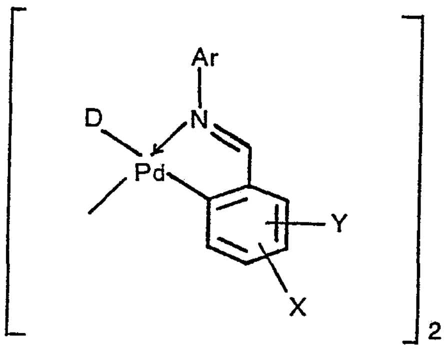

in which Ar is an inert aryl residue and D is an anion, with a Grignard reagent of the structure:

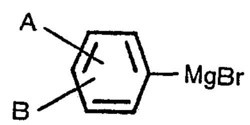

or the corresponding substituted phenyllithium in the presence of six to eight molar equivalents of triphenylphosphine, wherein in the above formulas the substituents A, B, X and Y correspond to the substituents of the biphenyl-2-carboxaldehydes as defined in claim 1.

**Claim for the Contracting State AT:**

Process for preparing 3,5-dimethyl-4′-fluoro-1,1′-biphenyl-2-carboxaldehyde; 3,3′,5,5′-tetramethyl-1,1′-biphenyl-2-carboxaldehyde; 3′,4′-dichloro-3,5-dimethyl-1,1′-biphenyl-2-carboxaldehyde; and 5-chloro-4′-fluoro-3,3′-dimethyl-1,1′-biphenyl-2-carboxaldehyde; 3,3′,4′,5-tetrachloro-1,1′-biphenyl-2-carboxaldehyde, which comprises reacting a palladium complex of the structure:

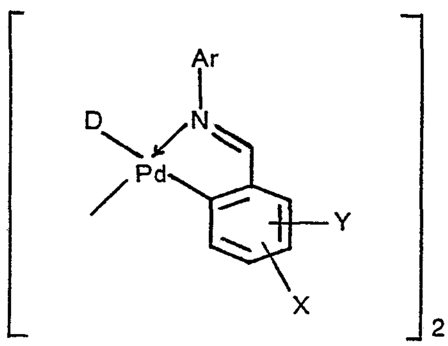

in which Ar is an inert aryl residue and D is an anion, with a Grignard reagent of the structure:

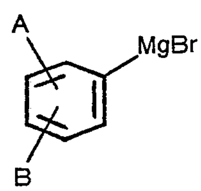

or the corresponding substituted phenyllithium in the presence of six to eight molar equivalents of triphenylphosphine, wherein in the above formulas the substituents A, B, X and Y correspond to the substituents of the biphenyl-2-carboxaldehydes as defined above.

**Patentansprüche für die Vertragsstaaten BE CH DE FR GB IT LI LU NL SE:**

1. Eine Verbindung, ausgewählt aus: 3,5-Dimethyl-4′-fluor-1,1′-biphenyl-2-carboxaldehyd; 3,3′,5,5′-Tetramethyl-1,1′-biphenyl-2-carboxaldehyd; 3′,4′-Dichlor-3,5-dimethyl-1,1′-biphenyl-2-carboxaldehyd; 5-Chlor-4′-fluor-3,3′-dimethyl-1,1′-biphenyl-2-carboxaldehyd; und 3,3′,4′,5-Tetrachlor-1,1′-biphenyl-2-carboxaldehyd.

2. Verfahren zur Herstellung eines Biphenyl-2-carboxaldehyds nach Anspruch 1, welches die Umsetzung eines Palladiumkomplexes der Struktur:

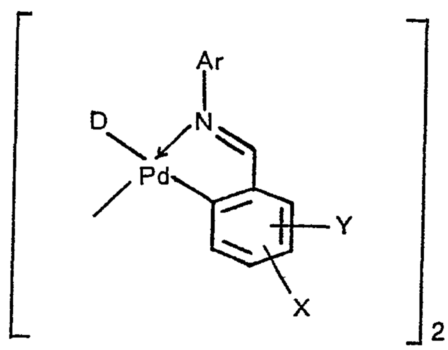

in welcher Ar ein inerter Arylrest ist und D ein Anion ist, mit einem Grignard-Reagens der Struktur:

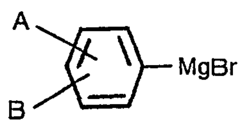

oder dem entsprechenden substituierten Phenyllithium, in Gegenwart von sechs bis acht Moläquivalenten Triphenylphosphin, umfaßt, wobei in den obigen Formeln die Substituenten A, B, X und Y den Substituenten der Biphenyl-2-carboxaldehyde, wie sie in Anspruch 1 definiert sind, entsprechen.

**Patentanspruch für den Vertragsstaat AT:**

Verfahren zur Herstellung von 3,5-Dimethyl-4′-fluor-1,1′-biphenyl-2-carboxaldehyd; 3,3′,5,5′-Tetramethyl-1,1′-biphenyl-2-carboxaldehyd; 3′,4′-Dichlor-3,5-dimethyl-1,1′-biphenyl-2-carboxaldehyd; 5-Chlor-4′-fluor-3,3′-dimethyl-1,1′-biphenyl-2-carboxaldehyd; und 3,3′,4′,5-Tetrachlor-1,1′-biphenyl-2-carboxaldehyd, welches die Umsetzung eines Palladiumkomplexes der Struktur

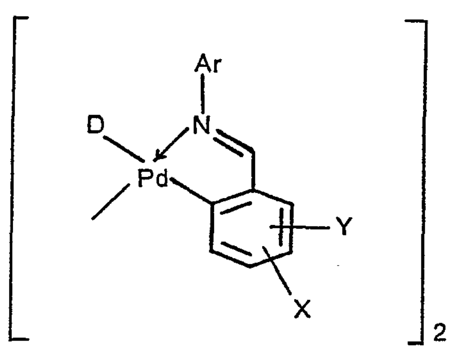

in welcher Ar ein inerter Arylrest ist und D ein Anion ist, mit einem Grignard-Reagens der Struktur:

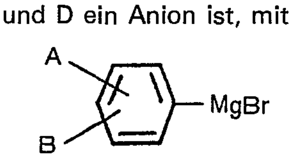

oder dem entsprechenden substituierten Phenyllithium, in Gegenwart von sechs bis acht Moläquivalenten Triphenylphosphin, umfaßt, wobei in den obigen Formeln die Substituenten A, B, X und Y den Substituenten der Biphenyl-2-carboxaldehyde, wie sie oben definiert sind, entsprechen.

**Revendications pour les Etats Contractants BE CH DE FR GB IT LI LU NL SE:**

1. Composé choisi parmi le diméthyl-3,5 fluoro-4′ biphényl-1,1′ carboxaldéhyde-2; le tétra-

méthyl-3,3',5,5' biphényl-1,1' carboxaldéhyde-2; le dichloro-3',4' diméthyl-3,5 biphényl-1,1' carboxaldéhyde-2; le chloro-5 fluoro-4' diméthyl-3,3' biphényl-1,1' carboxaldéhyde-2; le tétrachloro-3,3',4',5 biphényl-1,1' carboxaldéhyde-2.

2. Procédé de préparation d'un biphénylcarboxaldéhyde-2 selon la revendication 1, caractérisé en ce qu'il comprend la réaction d'un complexe au palladium de structure:

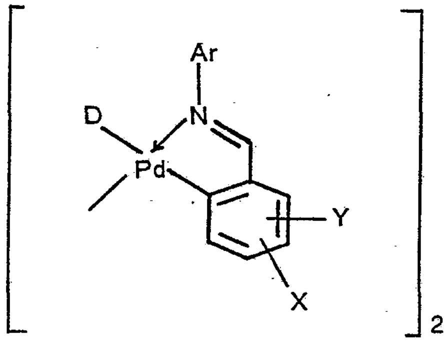

dans laquelle Ar est un résidu aryle inerte et D est un anion, avec un réactif de Grignard de structure:

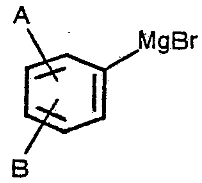

ou le phényllithium substitué correspondant en présence de six à huit équivalents molaires de triphénylphosphine, dans lequel, dans les formules précédentes, les substituants A, B, X et Y correspondent aux substituants des biphénylcarboxaldéhydes définis dans la revendication 1.

**Revendication pour l'Etat Contractant AT:**

Procédé de préparation du diméthyl-3,5 fluoro-4' biphényl-1,1' carboxaldéhyde-2; du tétraméthyl-3,3',5,5' biphényl-1,1' carboxaldéhyde-2; du dichloro-3',4' diméthyl-3,5 biphényl-1,1' carboxaldéhyde-2; du chloro-5 fluoro-4' diméthyl-3,3' biphényl-1,1' carboxaldéhyde-2; et du tétrachloro-3,3',4',5 biphényl-1,1' carboxaldéhyde-2, caractérisé en ce qu'il comprend la réaction d'un complexe au palladium de structure:

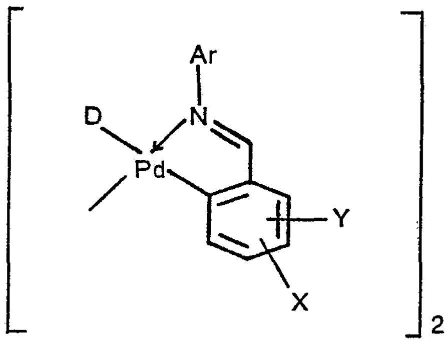

dans laquelle Ar est un résidu aryle inerte et D est un anion, avec un réactif de Grignard de structure:

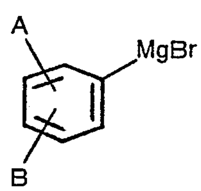

ou le phényl-lithium substitué correspondant en présence de six à huit équivalents molaires de triphénylphosphine, dans lequel, dans les formules précédentes, les substituants A, B, X et Y correspondent aux substituants des biphénylcarboxaldéhydes définis ci-dessus.